# FASCICULE DE BREVET EUROPEEN

(11) **EP 0 743 985 B1**
(45) Date de publication et mention de la délivrance du brevet: **11.10.2000**
(21) Numéro de dépôt: 95901568.6
(22) Date de dépôt: 12.12.1994
(51) Int. Cl.: C12P 7/40

(54) **PROCEDE POUR LA PREPARATION DE CETONES CYCLIQUES**
Verfahren zur Herstellung von cyclischen Ketonen
METHOD FOR PREPARING CYCLIC KETONES

(43) Date de publication de la demande: 27.11.1996
(73) Titulaire: FIRMENICH SA, 1211 Genève 8 (CH)
(72) Inventeur: WHITEHEAD, Ian, Michael, CH-1233 Bernex (CH)
(74) Mandataire: Salvaterra-Garcia, Maria de Lurdes
(86) Numéro de dépôt international: IB9400409
(87) Numéro de publication internationale: WO9618742

(56) Documents cités:
- EP-A- 0 354 000
- DD-A- 216 734
- FR-A- 1 417 723
- GB-A- 1 286 266

## Description

### DOMAINE TECHNIQUE

La présente invention a trait au domaine de la synthèse bioorganique. Elle concerne, plus particulièrement, un procédé pour la préparation de cétones cycliques de formule 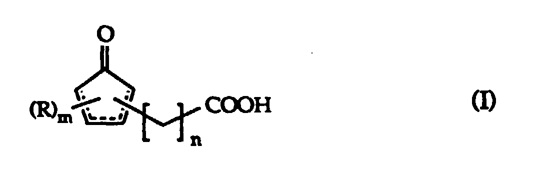 pouvant avoir une double liaison dans l'une des positions indiquées par les lignes pointillées et dans laquelle m représente un nombre enfler de 0 à 3 et n un nombre entier de 0 à 10, le ou les symboles R représentent l'hydrogène ou des radicaux alkyles identiques ou différents, saturés ou insaturés, linéaires ou ramifiés, ayant de 1 à 6 atomes de carbone, chacun des groupes substituants pouvant se trouver dans n'importe quelle position libre du cycle, le procédé étant caractérisé en ce qu'on ajoute un substrat contenant un ou plusieurs dérivés cycliques carboxyliques de formule 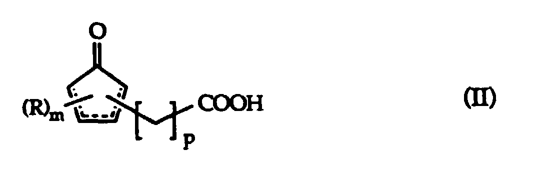 dans laquelle les lignes pointillées et les symboles R et m ont le sens indiqué à la formule (I), p ≥ n+2 et est défini comme étant un nombre entier pair lorsque n est pair et impair lorsque n est impair, à une culture d'un microorganisme capable d'effectuer l'oxydation en β de la chaîne d'acide gras desdits dérivés, pour fournir au moins une des cétones désirées, que l'on extrait ensuite du milieu réactionnel.

### TECHNIQUE ANTERIEURE

On connaît de l'art antérieur des procédés microbiologiques qui font appel à des oxydations en β de substrats dérivés d'acides gras. C'est ainsi que l'on décrit, par exemple dans US 5,168,054, un procédé de ce type pour la préparation de lactones, à partir de dérivés des acides linolénique, linoléique et oléique. Cependant, à note connaissance, de tels procédés n'ont jamais été appliqués à des substrats cycliques carboxyliques tels que ceux utilisés à présent.

### EXPOSE DE L'INVENTION

Or, nous avons découvert que le procédé selon la présente invention permettait de préparer une grande variété de dérivés des cyclopenténones et de la cyclopentanone, et ceci dans des conditions parfaitement applicables à l'échelle industrielle et avec des rendements très avantageux. Le procédé de l'invention se révèle être, en fait, d'une application très générale, aussi bien en ce qui concerne la nature des substrats, qu'en ce qui a trait à la variété de microorganismes pouvant être utilisés.

En effet, il a été constaté que l'on pouvait utiliser, avec égal succès, des substrats dans lesquels la chaîne d'acide gras se trouvait aussi bien en position α qu'en position β du cycle, par rapport au groupe cétonique, et que ledit cycle pouvait encore porter d'autres substituants R dont la nature est identique ou différente.

C'est ainsi que, selon un mode d'exécution préféré de l'invention, qui s'est révélé particulièrement avantageux, on utilise un substrat de formule 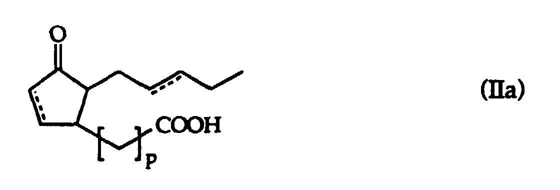 dans laquelle les lignes pointillées indiquent l'emplacement d'une liaison simple ou double et p ≥ n+2 et est impair. Selon ce mode préféré, on peut ainsi obtenir des composés de formule 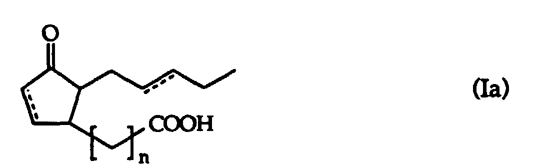 n étant un nombre entier impair, parmi lesquels on trouve des dérivés de la cyclopentanone qui sont des composés utiles pour la synthèse de substances odorantes. Par exemple, l'acide 3-oxo-2-(2-pentényl)-1-cyclopentaneacétique, ou acide jasmonique, est un précurseur du jasmonate de méthyle, un composé bien apprécié en parfumerie et un composant naturel de l'huile essentielle de jasmin.

De même, l'acide 3-oxo-2-pentyl-1-cyclopentaneacétique, ou acide dihydrojasmonique, est un précurseur de l'Hédione® (3-oxo-2-pentyl-1-cyclopentaneacétate de méthyle ; origine; Firmenich SA, Genève, Suisse), un ingrédient parfumant très prisé pour son odeur florale, jasminée et son effet olfactif exaltant.

En plus de leur intérêt pour la parfumerie, ces acides et leurs esters méthyliques sont également utiles dans l'industrie des arômes, pour la préparation de produits comestibles, et il existe donc un besoin réel de disposer d'un procédé bioorganique, permettant de les obtenir par voie microbiologique.

On sait, par ailleurs, que plusieurs variétés de plantes sont capables de les produire et que l'acide jasmonique et le jasmonate de méthyle en particulier jouent un rôle important dans le métabolisme de ces plantes et ont notamment une action régulatrice de leur croissance. Ceci a suscité l'intérêt de plusieurs chercheurs qui se sont attelés à étudier leur biosynthèse, effectuée par le biais des enzymes présentes dans les tissus végétaux correspondants (voir par exemple, B.A. Vick et al., Plant Physiol. 1984, 75, 458-61). Il est clair, cependant, que l'isolation des composés susmentionnés à partir de ces tissus végétaux, n'est pas une synthèse économiquement viable à large échelle.

GB-A-1286266 et DD-A-216734 divulguent la production de l'acide jasmonique et ses analogues par voie biosynthetique.

Or, le procédé selon la présente invention apporte justement une contribution nouvelle au problème posé par la synthèse bioorganique de ces composés. Grâce à ce procédé, les composés de formules (I) et (Ia) peuvent être préparés avec des rendements excellents, à l'aide d'une grande variété de microorganismes, dont on ne connaissait pas jusqu'ici la capacité de métaboliser les substrats de formule (II), et plus particulièrement les substrats de formule (IIa) qui permettent d'obtenir les acides jasmonique et dihydrojasmonique. Rien ne laissait donc prévoir qu'un procédé tel que revendiqué à présent ouvrirait une voie nouvelle et générale pour la préparation de dérivés des cydopenténones et de la cydopentanone, laquelle se révèle particulièrement utile pour la synthèse d'ingrédients de grand intérêt pour la parfumerie et l'industrie alimentaire.

En plus, il a été constaté que certains modes d'exécution du procédé de l'invention favorisaient l'obtention d'isomères déterminés des composés (Ia). En effet, de par leur structure, ces composés peuvent se présenter sous deux formes stéréoisomères de configuration cis et trans, chacune desquelles possède deux énantiomères.

Il convient néanmoins de noter que, si l'obtention des acides jasmonique et dihydrojasmonique constitue un des buts principaux de l'invention, il n'en reste pas moins que le procédé décrit à présent est d'une application bien plus vaste et permet d'obtenir une grande variété de dérivés des cyclopenténones et de la cyclopentanone.

Par exemple, selon un autre mode d'exécution de l'invention, on utilise en tant que substrat un composé de formule 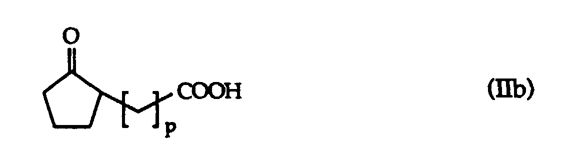 dans laquelle p a le sens indiqué à la formule (II), pour obtenir l'acide 2-oxo-1-cyclopentaneacétique et ses homologues supérieurs.

Il ne semble, en effet, exister aucune limitation à la nature des substrats qui peuvent être transformés selon le procédé décrit ci-dessus. C'est ainsi que l'on peut également utiliser des substrats qui possèdent des chaînes d'acide gras encore plus longues que celles citées plus haut, et que ces chaînes peuvent éventuellement être insaturées et même substituées par des radicaux alkyles inférieurs, notamment des radicaux méthyle et éthyle. Il convient de noter que lorsque le substrat possède une chaîne d'acide gras très longue, le procédé selon l'invention permet de le convertir en plusieurs homologues inférieurs, qui se forment en séquence réactionnelle et qui peuvent être ensuite convertis eux-mêmes en homologues inférieurs jusqu'à formation des composés (I) correspondants où n=0 ou 1. Ainsi, le produit de l'oxydation peut être constitué par un ou plusieurs de ces métabolites de la séquence réactionnelle, suivant le temps de réaction et les caractéristiques cinétiques des oxydations en β successives. Le cas échéant, une fois le mélange réactionnel extrait du milieu, les différents composants de ce mélange peuvent être séparés par les méthodes usuelles, telles que chromatographie ou distillation. Alternativement, et selon la nature du produit que l'on désire obtenir, on laisse le microorganisme agir jusqu'à ce que tous les métabolites intermédiaires aient été transformés à la fois et on recueille essentiellement le dernier produit de la séquence réactionnelle.

C'est ainsi que l'on peut obtenir par exemple essentiellement de l'acide jasmonique ou 2-(2-pentényl)-3-oxo-1-cydopentaneacétique à partir de l'acide 3-oxo-2-[2-pentényl]-1-cyclopentaneoctanoïque, un composé d'occurrence naturelle dans certaines plantes, ou encore à partir de l'un de ses homologues inférieurs. Ces transformations sont décrites en détail dans les exemples présentés plus loin.

Parmi les microorganismes pouvant être utilisés selon l'invention pour effectuer l'oxydation en β des substrats (II), ceux du genre *Saccharomyces* ou *Rhodococcus* se sont révélés d'un usage particulièrement avantageux, notamment pour la préparation des acides jasmonique et dihydrojasmonique.

On peut citer par ailleurs, à titre de microorganismes dont l'emploi est préféré selon l'invention, ceux sélectionnés dans le groupe constitué par *Rhodococcus rhodochorus, Rhodococcus erytropolis, Rhodococcus sp., Nocardia calcarea, Arthrobacter petroleophagus, Arthrobacter artrocyanus, Arthrobacter ureafaciens, Aspergillus niger, Saccharomyces cerivisae, Mycobacterium phlei, Streptomyces viridosporus, Streptomyces rosechromogenus, Streptomyces bacilliaris, Cylindrocarpon candidum, Escherichia coli, Hansenula polymorpha, Pseudomonas Sp., Serratia marcesens* et *Aspergillus oryzae.* Ces microorganismes sont disponibles auprès des organisations de dépôt de notoriété internationale. Dans le cas particulier des *Saccharomyces*, on peut les acheter auprès d'entreprises spécialisées, lesquelles fournissent souvent des espèces de culture locale, notamment issues des industries de la bière, du vin ou encore boulangère. Toutes ces espèces conviennent parfaitement pour le procédé selon l'invention. La culture de ces microorganismes est obtenue selon des méthodes classiques. Leur croissance préalable est effectuée dans des milieux nutritifs de type courant et dans les conditions usuelles, soit en phase stationnaire, soit sous agitation.

Les cellules sont ensuite isolées du milieu de croissance, par exemple par centrifugation, et suspendues en milieu aqueux contenant les substrats susmentionnés, généralement dépourvu de toute autre source nutritive, à des températures comprises de préférence entre 20 et 35°C, pendant des périodes de temps variables mais relativement courtes, comprises typiquement entre 24 et 72 heures, dans des conditions aérobiques. Le pH pendant la réaction sera maintenu de préférence entre 5 et 10.

Selon un mode d'exécution particulier de l'invention, avant d'ajouter le substrat à cette culture du microorganisme, on prévoit une première étape de préparation à travers une période d'aération de la culture, qui consiste à la suspendre dans de l'eau, dans des conditions aérobiques et sous agitation, à une température comprise entre 15 et 30°C, pendant suffisamment de temps pour garantir que toute source nutritive, d'origine endogène ou exogène, a été entièrement consommée avant l'addition des substrats de formule (II). Une telle étape de préparation est particulièrement appropriée lorsque la culture du microorganisme, en résultat des conditions dans lesquelles sa croissance a eu lieu, possède une source nutritive résiduelle, notamment de carbohydrates, comme c'est typiquement le cas des *Saccharomyces* par exemple.

Le substrat est alors ajouté à la culture du microorganisme ainsi préparée, l'addition ayant lieu de préférence en l'absence de toute autre source nutritive, dans les conditions citées plus haut.

Les cellules sont ensuite séparées du milieu réactionnel par centrifugation ou ultrafiltration et la solution aqueuse ainsi obtenue acidifiée et extraite à plusieurs reprises à l'aide d'un solvant approprié, par exemple de l'éther diéthylique. Les phases organiques réunies sont ensuite traitées de façon courante et, si désiré, leurs composants (I) séparés par chromatographie.

Il convient de noter que les acides ainsi obtenus peuvent ensuite être facilement estérifiés par voie chimique ou enzymatique pour former les esters correspondants.

Les substrats de formule (II) sont des composés d'origine commerciale ou peuvent être aisément préparés à partir de produits commerciaux. Par exemple, les substrats dérivés de la cyclopentanone peuvent être préparés de façon classique à l'aide d'additions sur la cyclopent-2-én-1-one, ou des dérivés de celle-ci, selon le schéma général que voici : 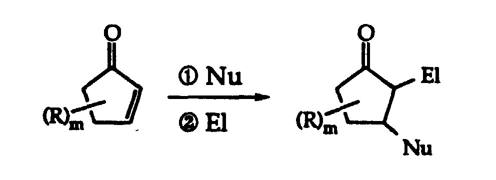
R et m définis à la formule (I)
El - groupe électrophyle
Nu - groupe nucléophyle
la chaîne d'acide gras étant introduite sous forme d'un groupe qui agira en tant que nucléophile ou électrophyle, suivant la position du cycle dans laquelle on désire l'introduire et les conditions de l'addition. Par exemple, les substrats de formule (IIa), à cycle saturé, peuvent être préparés selon le schéma que voici : 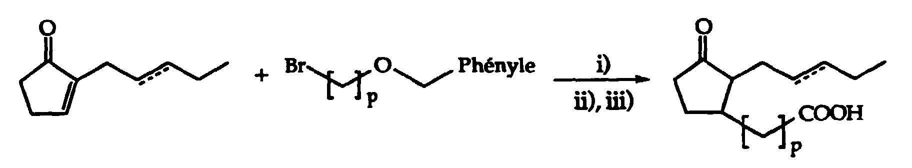
i) Mg, (C₂H₅)₂O, CuBr.(CH₃)₂S, - 40°C
ii) (CH₃)₃SiI, CH₂Cl₂, t.a., HCl aq., THF, t.a.
iii) H₂CrO₄ aq., H₂SO₄, acétone, t.a.
Dans ce schéma, la ligne pointillée et p ont le sens indiqué à la formule (IIa). La cyclopenténone de départ est un composé connu (voir, par exemple, EP 110 142) et le réactif bromé peut être obtenu de façon classique et comme il est décrit plus loin, à partir de diols d'origine commerciale.

Pour ce qui est des substrats de formule (II) possédant une double liaison dans le cycle, ils peuvent être obtenus de façon analogue à celle décrite par exemple par P.A. Grieco et al. dans J. Org. Chem. 1989, 54, 6008-6010, et tel que représenté ci-après : 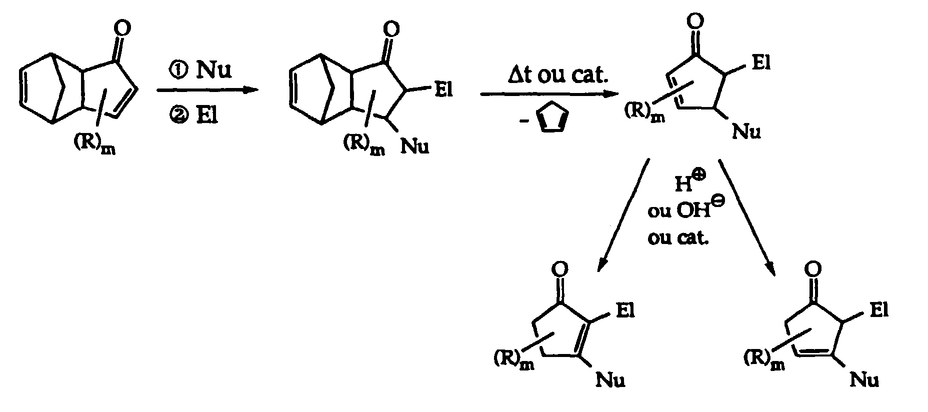
R et m définis à la formule (I)
El - groupe électrophyle El - groupe électrophyle
Nu - poupe nucléophyle
Les conditions de ces réactions de type classique sont décrites en détail dans les exemples présentés plus loin.

Bien entendu, les substrats de formule (II) peuvent être utilisés sous forme de mélanges racémiques de stéréoisomères de configuration cis et trans, sous forme des racémates de configuration cis ou trans, ou encore sous forme de l'un des quatre diastéréomères à l'état pur. Il a été observé que certains microorganismes, lorsque mis en contact avec des substrats racémiques, contenant des mélanges d'isomères cis et trans, favorisaient la formation de certains produits finals chiraux. Les détails de ces transformations sont présentés dans les exemples ci-après.

Le procédé de l'invention sera maintenant décrit plus en détail à l'aide de ces exemples dans lesquels les températures sont indiquées en degrés centigrades et les abréviations ont le sens usuel dans l'art. Dans les tableaux, les quantités des produits obtenus sont exprimées en pourcentage résultant des analyses chromatographiques, par rapport à un standard interne.

### MANIERES DE REALISER L'INVENTION

### Exemple 1

### METHODE GENERALE I

On a placé dans un ballon à 5 cols (200 ml), equipé d'une turbine de type Medimix®, d'une cellule de mesure du pH, d'une entrée d'air, d'un réfrigérant et d'une sortie d'échantillonnage, la culture du microorganisme (10 g de biomasse humide) et 50 ml d'eau déminéralisée. La suspension de cellules a été aérée (1 v/v/m) et agitée (1,000 rpm) pendant la nuit à 30°. Le pH de la suspension a ensuite été ajusté à 5,5 et une solution du substrat à utiliser, dans ce cas l'acide 3-oxo-2-pentyl-1-cyclopentaneoctanoïque (100 mg, 0,34 mmole, 2 g/l; voir préparation plus loin) dans l'eau (10 ml) a été ajoutée. La réaction a pu être suivie en prenant des aliquots (1 ml) du mélange à intervalles réguliers. Ces échantillons ont été centrifugés dans des tubes plastiques de type Eppendorf pendant 2 minutes pour précipiter les cellules. Un aliquot (0,45 ml) de la solution transparente surnageante a ensuite été acidifié avec HCl 5M (0,05-0,1 ml) et extrait avec éther diéthylique (2 fois, volumes identiques). Les extraits organiques ont été combinés, séchés sous azote et traités avec diazométhane pour former les esters méthyliques des acides contenus dans le produit de la réaction. Ce mélange d'esters a ensuite été analysé par chromatographie en phase gazeuse.

A la fin de 24 h de réaction, le mélange réactionnel a été centrifugé (20 min, à 10,000 rpm) pour précipiter les cellules et le liquide surnageant décanté. Afin de laver les cellules, on les a suspendues à nouveau dans 50 ml d'eau et on a agité pendant 30 min. On a recentrifugé pour obtenir un deuxième liquide surnageant qui a été combiné avec le premier et dont le pH a été ajusté à 12 avec NaOH (10%). La solution résultante a été extraite avec de l'éther diéthylique (3 fois, volumes identiques) pour séparer la fraction neutre.

Le pH a été ensuite porté à 2 avec HCl (5 M) et la fraction acide récupérée par extraction. Les extraits organiques combinés ont été séchés sur Na₂SO₄ et le solvant enlevé sous vide. Un aliquot a ensuite été méthylé à l'aide de diazométhane et le produit ainsi obtenu a été analysé par chromatographie en phase gazeuse [colonne de type SPB5, 30 m de longueur, 0,32 mm de diamètre interne, 50°(0')-230°(5') à 10°/min; échantillon dissous dans 0,1 ml de diisopropyléther contenant 1 g/l de myristate de méthyle comme standard interne - temps de rétention 13,91 minutes].

Lors d'un essai effectué à une échelle 5 fois plus grande, en utilisant des *Saccharomyces cerevisiae* (origine : Hefe, Schweiz, AG, 9507 Stettfurt, Switzerland), on a isolé et identifié les composés suivants [CG-SM : M/Z (% par rapport standard interne)]:

### 1. 3-oxo-2-pentyl-1-cyclopentaneacétate de méthyle

- isomère trans :: temps de rétention - 13,18 min
- CG-SM :: 226 (3) [M⁺], 195 (2) [M-OCH₃⁺], 156 (35) [M-C₅H₁₁+H⁺], 153 (31) [M-CH₂-COOCH₃⁺], 109 (5), 96(10), 83(100) [C₅H₆O+H⁺], 82 (24) [C₅H₆O⁺], 55(13)
- isomère cis :: temps de rétention - 13,46 min
- CG-SM :: 226 (5) [M+], 156 (25) [M-C₅H₁₁+H⁺], 153 (29) [M-CH₂COOCH₃⁺], 109 (5), 103 (15), 96 (11), 83 (100) [C₅H₆O+H⁺], 82 (24) [C₅H₆O⁺], 55 (19)

### 2. 3-oxo-2-pentyl-1-cyclopentanebutanoate de méthyle

- isomère trans :: temps de rétention - 15,53 min
- CG-SM :: 254 (1) [M⁺], 184 (13) [M-C₅H₁₁+H⁺], 153 (29) [M-(CH₂)₃COOCH₃⁺], 109 (4), 97 (5), 83 (100) [C₅H₆O+H⁺], 82 (30) [C₅H₆O⁺], 55 (10)
- isomère cis :: temps de rétention - 15,72 min
- CG-SM :: 254 (1) [M⁺], 184 (13) [M-C₅H₁₁+H⁺], 153 (29) [M-(CH₂)₃COOCH₃⁺], 109 (4), 97 (5), 83 (100) [C₅H₆O+H⁺], 82 (29) [C₅H₆O⁺], 55 (10)

### 3. 3-oxo-2-pentyl-1-cyclopentanehexanoate de méthyle

- isomère trans :: temps de rétention - 17,42 min
- CG-SM :: 282 (1) [M⁺], 251 (4) [M-OCH₃⁺], 212 (13) [M-C₅H₁₁+H⁺], 153 (18) [M-(CH₂)₅COOCH₃⁺], 130 (29), 83 (100) [C₅H₆O+H⁺], 82 (8) [C₅H₆O⁺], 55 (11)
- isomère cis :: temps de rétention - 17,65 min

### 4. 3-oxo-2-pentyl-1-cyclopentaneoctanoate de méthyle

- isomère trans :: temps de rétention - 19,35 min
- CG-SM :: 310 (1) [M⁺], 279 (2) [M-OCH₃⁺], 240 (11) [M-C₅H₁₁+H⁺], 153 (31) [M-(CH₂)₇COOCH₃⁺], 83 (100) [C₅H₆O+H⁺], 82 (38) [C₅H₆O⁺], 55 (10)

### PREPARATION DU PRODUIT DE DEPART

Le produit de départ dans la réaction décrite ci-dessus, à savoir l'acide 3-oxo-2-pentyl-1-cyclopentaneoctanoïque, a été préparé ainsi.

Une solution de 1,8-octanediol (15 g, 0,1 mole) dans le toluène (235 ml) et le THF (tétrahydrofurane, 15 ml) a été ajoutée goutte à goutte à une pâte de NaH (dispersion dans l'huile 65-70%, 5,3 g, 0,15 mole) dans le toluène (50 ml), maintenue sous agitation et N₂, à température ambiante. Le mélange a été chauffé à reflux pendant 60 h et on a alors ajouté du chlorure de benzyle (33 g, 0,26 mole) et maintenu à reflux pendant encore 60 h. Le mélange réactionnel refroidi a été versé sur NH₄Cl aq. sat., refroidi et extrait à l'éther. Les phases organiques combinées ont été lavées avec NaCl aq. sat., séchées sur Na₂SO₄ anhydre et concentrées sous vide pour fournir une huile jaune pâle (24,5 g). Après chromatographie sur colonne de silice (350 g), avec cyclohexane/acétate d'éthyle 4:1 comme éluant, suivie de distillation au four à boules sous vide, on a obtenu le 8-benzyloxyoctan-1-ol pur, sous forme d'une huile jaune pâle (11,9 g, rend. 49%).
P.éb.: 200-220° (bain) / 4 Pa
Rf (cyclohexane/acét. éthyle 7:3) 0,36
IR (CDCl₃) : 3450 (large), 3010, 2934, 2858, 1454, 1095 cm⁻¹
RMN (¹H, 360MHz, D₂O) : 1,25-1,45(8H); 1,45-1,70(4H) ; 3,46(t,J=7Hz,2H) ; 3,60(t,J=7Hz,2H) ; 4,50(s,2H) ; 7,23-7,38(5H) δ ppm
RMN (¹³C): 138,6(s); 128,3(d); 127,6(d); 127,5(d); 72,9(t); 70,5(t); 62,9(t); 32,7(t); 29,7(t); 29,4(2t) ; 28,1(t); 25,7(t) δ ppm
SM : 236 (2,M⁺), 107(59), 91(100)

A une solution agitée de ce composé (11,6 g, 0,049 mole) et de pyridine (9,8 g, 0,12 mole) dans CH₂Cl₂ (100 ml), on a ajouté par portions, à température ambiante (t.a.) et sous N₂, du chlorure de tosyle (12,8 g, 0,067mole). Après 20 h à t.a., le mélange a été versé sur HCl aq. à 5%, refroidi et extrait (CH₂Cl₂). La phase organique a été lavée avec NaHCO₃ aq. sat. et saumure, séché sur Na₂SO₄ anhydre et concentré. L'huile semi-cristalline résiduelle (19,2 g ; tosylate brut du benzyloxyoctanol susmentionné) a été reprise à l'acétone (400 ml) et on a ajouté du LiBr (10,9 g, 0,126 mole). Le mélange sous agitation a alors été porté à reflux pendant 90 min, refroidi à t.a. et filtré. Le filtrat a été concentré et dissous dans l'éther (150 ml). Cette phase organique a alors été lavée successivement avec H₂O et NaCl aq. sat., et séchée sur Na₂SO₄ anhydre. La concentration sous vide (80°/8 Pa) a fourni le 1-benzyloxy-8-bromooctane sous forme d'une huile jaune pâle (12,4 g; rend. 84%).
IR (CHCl₃) : 3011, 2934, 2858, 1454, 1364, 1098 cm⁻¹
RMN (¹H, 360MHz) : 1,25-1,50(8H) ; 1,60(m,2H) ; 1,83(m,2H) ; 3,39(t,J=7Hz,2H) ; 3,46(t,J=7Hz,2H) ; 4,50(s,2H) ; 7,22-7,37(5H) δ ppm
RMN (¹³C) : 138,7(s) ; 128,3(d) ; 127,6(d) ; 127,5(d) ; 72,9(t) ; 70,4(t) ; 33,9(t) ; 32,8(t) ; 29,7(t) ; 29,3(t) ; 28,7(t) ; 28,1(t) ; 26,1(t) δ ppm
SM : 298 (11,M⁺), 207(52), 188(17), 147(16), 109(22), 9(100)

Une solution de ce bromooctane (11 g, 0,037 mole) dans l'éther (36 ml) a été ajoutée goutte à goutte à une pâte agitée de tournures de Mg (0,91 g, 0,037 mole) dans l'éther (4 ml), à t.a. et sous N₂. Le réactif de Grignard résultant a été chauffé à reflux pendant 30 min, ensuite refroidi et ajouté goutte à goutte à une pâte de CuBr.(CH₃)₂S (3,2 g, 0,015 mole) dans l'éther (30 ml), sous agitation et à - 44°. Après 3 min, une solution de 2-pentylcyclopent-2-én-1-one (4,7 g, 0,028 mole ; voir EP 110 142, par exemple) dans l'éther (15 ml) a été ajoutée goutte à goutte pendant 30 min, à - 44°. Après encore 15 min à - 40°, on a laissé la température monter à 0° pendant 1 h et ensuite versé le mélange sur NH₄Cl aq. refroidi. L'extraction à l'éther a fourni une phase organique qui a été successivement lavée avec H₂O et NaCl aq. sat. et séchée sur Na₂SO₄ anhydre. La concentration sous vide a fourni une huile verte semi-cristalline (12,7 g) qui a été purifiée par chromatographie [SiO₂ (280 g); éluant : cyclohexane/acét. éthyle 19:1)] pour fournir la 3-(8'-benzyloxyoct-1'-yl)-2-pentylcyclopentan-1-one (mélange trans/cis 12:1) sous forme d'une huile jaune (3,1 g; rend. 30%) qui a été séchée à 100°/7,9 Pa.
R_{f} (cyclohexane/acét. éthyle 9:1) 0,43
IR (CHCl₃) : 3011, 2930, 2857, 1732, 1455, 1098 cm⁻¹
RMN (¹H, 360MHz) : 0,88(t, J=7Hz, 3H) ; 1,20-2,40(28H) ; 3,47(t, J=7Hz, 2H) ; 4,50(s, 2H) ; 7,23-7,37(5H) δ ppm
RMN (¹³C) : 221,5(s) ; 138,7(s) ; 128,3(d) ; 127,6(d) ; 127,5(d) ; 72,9(t) ; 70,5(t) ; 55,1(d) ; 41,6(d) ; 37,9(t) ; 34,8(t) ; 32,2(t) ; 29,8(t) ; 29,6(t) ; 29,5(t) ; 28,1(t) ; 27,1(t) ; 26,6(t) ; 26,2(t) ; 22,5(t) ; 14,1(q) δ ppm
SM : 372 (6,M⁺), 243(6), 196(8), 173(14), 153(35), 91(100), 83(92)

Une solution de cette cyclopentanone (3 g, 8,05 mmole ; trans/cis 12:1) dans l'éthanol (30 ml) contenant Pd-C à 10% (0,24 g), a été hydrogénolysée à t.a. pendant 4 h. Après filtration (Hyflo®), concentration sous vide et distillation au four à boules, on a obtenu la 3-(8'-hydroxy-1'-octyl)-2-pentylcyclopentan-1-one (mélange trans/cis 12:1) sous forme d'une huile jaune pâle (2,1 g ; rend. 92%).
P. éb. : 230-240° (bain) / 5,3 Pa
IR (CHCl₃) : 3437 (large), 2930, 2857, 1732, 1456, 1160, 1051 cm⁻¹
RMN (¹H, 360MHz, D₂O) : 0,88(t,3H) ; 1,20-2,00(24H) ; 2,00-2,36(4H) ; 3,65(t,J=7Hz,2H) δ ppm
RMN (¹³C) : 221,6(s) ; 63,0(t) ; 53,1(d) ; 41,6(d) ; 37,9(t) ; 34,8(t) ; 32,8(t) ; 32,2(t) ; 29,8(t) ; 29,6(t) ; 29,4(t) ; 28,1(t) ; 27,1(2t) ; 26,6(t) ; 25,8(t) ; 22,5(t) ; 14,1(q) δ ppm
SM : 282 (0,M⁺), 212(4), 153(17), 83(100)

Du réactif de Jones (H₂CrO₄/H₂SO₄ aq. ; 7,2 ml d'une solution 2,5 M) a été ajouté goutte à goutte à une solution agitée de cette dernière cyclopentanone (2 g, 7,1 mmole ; trans/cis 12:1) dans l'acétone (40 ml) à 20°, sous N₂. Après encore 40 min à 22-24°, le mélange a été versé dans l'eau et extrait à l'éther. La phase organique a été lavée successivement avec H₂O, NaCl aq. à 10% et NH₄Cl aq. sat, séchée sur Na₂SO₄ anhydre et concentrée sous vide. L'huile jaune résultante (2,1 g) a été purifiée par chromatographie [SiO₂ (100 g) ; éluant : cyclohexane/acét. éthyle 1:1,5)] pour fournir l'acide 3-oxo-2-pentyl-1-cyclopentaneoctanoïque désiré (trans/cis 12:1) sous forme d'une huile visqueuse jaune pâle (1,9 g ; rend. 90%).
IR (CHCl₃) : 3050 (large), 2931, 2858, 1732, 1710, 1460, 1160 cm⁻¹
RMN (¹H, 360MHz, D₂O) : 0,89(t, J=7Hz, 3H) ; 1,20-2,35(28H) ; 2,37(t, J=7Hz, 2H) δ ppm
RMN (¹³C) : 221,8(s) ; 179,6(s) ; 55,1(d) ; 41,6(d) ; 37,9(t) ; 34,8(t) ; 34,0(t) ; 32,2(t) ; 29,6(t) ; 29,2(t) ; 29,0(t) ; 28,1(2t) ; 27,1(t) ; 26,6(t) ; 24,7(t) ; 22,5(t) ; 14,1(q) δ ppm
SM : 297 (2,M⁺), 279(3), 226(9), 153(17), 83(100)

### Exemple 2

On a procédé selon la méthode générale I décrite à l'Exemple 1, en utilisant une variété d'organismes différents, cités dans le tableau ci-après. Dans ce tableau, les composés sont indiqués par le chiffre qui leur a été attribué à l'Exemple 1. Ces résultats ont trait au produit obtenu après 24 h de réaction, c'est-à-dire d'activité du microorganisme correspondant. Le rendement en composé 1 représente le % molaire par rapport au composé 4, basé sur la quantité de l'acide de départ correspondant (100 mg).

**Tableau I**

| | | Composé (% CG) | | | | |
|---|---|---|---|---|---|---|
| Microorganisme | Poids extrait acides (mg) | 1 | 2 | 3 | 4 | Rend. en composé 1 (% molaire) |
| *Serratia marcesens ATCC 8100* | 36,6 | 84,5 | 11,9 | 1,4 | 2,2 | 43,2 |
| *Aspergillus niger ATCC 16888* | 25,0 | 74,4 | 9,8 | 6,0 | 9,8 | 26,0 |
| *Escherichia coli ATCC 8677* | 35,8 | 49,0 | 23,2 | 4,8 | 23,1 | 35,8 |
| *Saccharomyces cerevisiae* | 35,7 | 31,8 | 54,0 | 9,1 | 5,0 | 15,9 |
| *Streptomyces viriosporus ATCC 39115* | 34,3 | 30,4 | 25,9 | 8,4 | 35,3 | 14,6 |
| *Hansenula polymorpha* | - | 13,0 | 52,7 | 8,7 | 25,5 | - |
| *Pseudomonas Sp. DSM 1650* | - | 0,0 | 2,4 | 2,5 | 95,1 | - |

Ce tableau montre que tous ces microorganismes ont effectué l'oxydation en β des acides 3-oxo-2-pentyl-1-cyclopentaneoctanoïque, -hexanoïque et -butanoïque. Les rendements en acide final, i.e. acide 3-oxo-2-pentyl-1-cyclopentaneacétique, et donc en composé 1 correspondant, peuvent bien entendu être nettement améliorés en prolongeant la réaction au-delà de 24 h. Par ailleurs, la procédure d'extraction décrite peut également être améliorée pour réduire les pertes en produits désirés à un minimum.

### Exemple 3

### METHODE GENERALE II

Le microorganisme à utiliser dans la réaction a été cultivé au préalable pendant trois jours dans un milieu contenant de l'amidon. A la fin des trois jours, tout l'amidon avait été consommé.

Les cellules ont été récoltées par centrifugation et une portion de la biomasse résultante (2 g), ainsi que 8 ml d'eau déminéralisée, ont été placés dans un ballon de type Erlenmeyer (50 ml) contenant le substrat à utiliser, dans le cas présent l'acide (Z)-3-oxo-2-(2-pentényl)-1-cyclopentaneoctanoïque (20 mg, 2 g/l; voir préparation plus loin). Le ballon a été scellé avec un bouchon d'ouate et placé dans un appareil à agitation orbitale pendant 24 h, à 30° et 140 rpm.

Les cellules ont ensuite été précipitées par centrifugation (10,000 rpm) pendant 15 min à t.a. Après avoir enlevé le liquide surnageant avec une pipette, les cellules ont été suspendues à nouveau dans de l'eau déminéralisée (10 ml) et ensuite centrifugées. Le liquide surnageant résultant a été combiné avec celui obtenu en premier lieu et le volume combiné ajusté à 25 ml avec l'eau déminéralisée. Cette solution a ensuite été amenée à pH 2 avec H₂SO₄ (10%) et extraite à l'éther diéthylique (1x50 ml et 1x25 ml). Les phases organiques combinées ont été séchées sur MgSO₄ anhydre. Le solvant a été enlevé sous courant d'azote et le produit pesé.

On a ensuite traité avec du diazométhane pour obtenir les esters méthyliques correspondants. Le produit ainsi obtenu a été analysé par chromatographie par rapport à un standard externe. Pour ce faire, on a préparé une solution dans 1 ml de méthyl pentadécanoate (5 g/l) et chromatographié cette solution [colonne SPB5, 30 m de longueur, 0,32 mm de diamètre interne, 80°(0')-230°(20') à 15°/min]. La quantité de chacun des composants a été estimée sur la base de la surface du pic correspondant, par rapport à celle du standard. En procédant comme il est décrit ci-dessus, lors d'une réaction effectuée avec *Saccharomyces cerevisiae* (origine : Suisse), les produits suivants ont été obtenus et identifiés.

### A. (Z)-3-oxo-2-(2-pentényl)-1-cyclopentaneacétate de méthyle

- isomère trans :: temps de rétention - 10,28 min
- CG-SM :: 224 (31) [M+], 206 (4) [M-H₂O+], 193 (10) [M-OCH₃⁺], 177 (6), 156 (23) [M-C₅H₉+H⁺], 151 (31) [M-CH₂COOCH₃+], 109 (24), 95 (30), 83 (100) [C₅H₆O+H⁺], 55 (37), 41 (73)
- isomère cis :: temps de rétention - 10,56 min
- CG-SM :: 224 (24) [M⁺], 206 (12) [M-H₂O⁺], 177 (13), 156 (15) [M-C₅H₉+H⁺], 151 (31) [M-CH₂COOCH₃⁺], 109(23), 95 (42), 83 (100) [C₅H₆O+H⁺], 55 (19), 41 (81)

### B. (Z)-3-oxo-2-(2-pentényl)-1-cyclopentanebutanoate de méthyle

- isomère trans :: temps de rétention - 12,74 min
- CG-SM:: 252 (17) [M⁺], 234 (7) [M-H₂O⁺], 184 (10) [M-C₅H₉+H⁺], 151 (59) [M-(CH₂)₃COOH₃⁺], 109 (22), 95 (35), 83 (100) [C₅H₆O+H⁺], 41 (67)
- isomère cis :: temps de rétention - 13,10 min
- CG-SM :: 252 (8) [M+], 234 (20) [M-H₂O⁺], 205 (10), 184 (9) [M-C₅H₉+H⁺], 151 (37) [M-(CH₂)₃COOH₃⁺], 109 (30), 95 (47), 83 (100) [C₅H₆O+H⁺], 41 (73)

### C. (Z)-3-oxo-2-(2-pentényl)-1-cyclopentanehexanoate de méthyle

- isomère trans :: temps de rétention - 16,37 min
- CG-SM :: 280 (7) [M⁺], 262 (8) [M-H₂O+], 212 (16) [M-C₅H₉+H⁺], 151 (39) [M-(CH₂)₅COOCH₃⁺], 95 (40), 83 (100) [C₅H₆O+H⁺], 82 (8) [C₅H₆O⁺], 41 (55)
- isomère cis:: temps de rétention - 17,10 min

### D. (Z)-3-oxo-2-(2-pentényl)-1-cyclopentaneoctanoate de méthyle

- isomère trans :: temps de rétention - 22,71 min
- CG-SM :: 308 (4) [M⁺], 290 (4) [M-H₂O⁺], 277 (7) [M-OCH₃⁺], 240 (18) [M-C₅H₉+H⁺], 151 (43) [M-(CH₂)₇COOCH₃⁺], 124 (35), 95 (48), 83 (100) [C₅H₆O+H⁺], 55 (43)
- isomère cis :: temps de rétention - 23,82 min
- CG-SM :: 308 (3) [M⁺], 290 (4) [M-H₂O⁺], 277 (5) [M-OCH₃^{**+**}], 240 (14) [M-C₅H₉+H⁺], 151 (37) [M-(CH₂)₇COOCH₃⁺], 124 (40), 95 (50), 83 (100) [C₅H₆O+H⁺], 55 (24)

Le produit de la réaction a également été analysé sur colonne chirale de type Megadex® diméthylpentyl-β-cydodextrine (10 m x 2,5 mm x 0,25µm d'épaisseur du film) en utilisant un programme de températures de 50°(1') - 130°(15'), chauffant 15°/min, et jusqu'à 220°(5'), chauffant 2°/min. Les temps de rétention de chacune des espèces chirales correspondant à chacun des composés A, B, C et D ci-dessus sont indiqués au tableau suivant.

**Tableau II**

| Isomère chiral | Temps de rétention (min) |
|---|---|
| (-)-trans-A | 17,61 |
| (+)-trans-A | 18,93 |
| (+)-cis-A | 20,29 |
| (-)-cis-A | 20,29 |
| (-)-trans-B | 33,78 |
| (+)-trans-B | 34,83 |
| (+)-cis-B | 35,86 |
| (-)-cis-B | 35,86 |
| (-)-trans-C | 44,57 |
| (+)-trans-C | 45,16 |
| (+)-cis-C | 46,32 |
| (-)-cis-C | 46,32 |
| (-)-trans-D | 53,7 - 54,1 |
| (+)-trans-D | 53,7 - 54,1 |
| (+)-cis-D | 55,21 |
| (-)-cis-D | 55,21 |

### PREPARATION DU PRODUIT DE DEPART

Le produit de départ dans la réaction décrite ci-dessus, à savoir l'acide (Z)-3-oxo-2-(2-pentényl)-1-cyclopentaneoctanoïque, a été préparé ainsi. Une solution de 1-benzyloxy-8-bromooctane (5 g, 0,015 mole ; voir Exemple 1) dans l'éther (15 ml) a été ajoutée goutte à goutte à une suspension agitée de tournures de Mg (0,38 g, 0,016 mole) dans l'éther (2 ml), à t.a. sous N₂. Le réactif de Grignard résultant a été chauffé à reflux pendant 30 min, ensuite refroidi et ajouté goutte à goutte à une pâte de CuBr.(CH₃)₂S (1,25 g, 6 mmole) et LiBr (1,9 g, 0,022 mole) dans le THF (15 ml), sous agitation et à - 44°. Après 3 min, une solution de (Z)-2-(2-pentényl)-cyclopent-2-én-1-one (1,65 g, 0,011 mole ; voir, par exemple, F. Näf et al., Helv. Chim. Acta 1978, 2524) et de chlorure de triméthylsilyle (2,8 ml, 0,022 mole) dans l'éther (15 ml) a été ajoutée goutte à goutte pendant 30 min, à - 44°. Après encore 15 min à - 40°, on a laissé la température monter à 0° pendant 1 h et ensuite versé le mélange sur NH₄Cl aq. refroidi. L'extraction à l'éther a fourni une phase organique qui a été successivement lavée avec H₂O et NaCl aq. sat. et séchée sur Na₂SO₄ anhydre. La concentration sous vide a fourni une huile brute (7 g) constituée par le triméthylsilyl énol éther de l'intermédiaire désiré, laquelle huile a été dissoute dans THF (25 ml) et agitée avec HCl aq. à 10% (1 ml) pendant 30 min. Après nouvelle extraction à l'éther, traitement et purification par chromatographie [SiO₂ (100 g) ; éluant : cyclohexane/acét. éthyle 19:1], on a obtenu la (Z)-3-(8'-benzyloxyoct-1'-yl)-2-(2-pentényl)-cyclopentan-1-one (mélange trans/cis 13:1) sous forme d'une huile jaune visqueuse (2,1 g ; rend. 52%) qui a été séchée à 50°/1,3 Pa.
RMN (¹H, 360MHz) : 4,96(t, J=7Hz, 3H) ; 1,20-2,40(24H) ; 7,47(t, J=7Hz, 2H) ; 4,50(s, 2H) ; 5,25(m, 1H) ; 5,42(m, 1H) ; 7,25-7,35(5H) δ ppm
RMN (¹³C) : 220,8(s) ; 138,7(s) ; 133,4(d) ; 128,3(d) ; 127,6(d) ; 127,5(d) ; 125,5(d) ; 72,9(t) ; 70,5(t) ; 55,1(d) ; 41,2(d) ; 38,1(t) ; 34,7(t) ; 29,8(t) ; 29,5(2t) ; 27,1(t) ; 26,2(t) ; 25,4(t) ; 20,6(t) ; 14,2(q) δ ppm
SM : 370 (1,M⁺), 302(10), 279(26), 261(9), 173(10), 151(30), 91(100), 83(25)

De l'iodure de triméthylsilyle a été ajouté via une seringue à une solution agitée de cette pentanone (2,1 g, 5,7 mmole ; trans/cis 13:1) dans CH₂Cl₂ (8 ml), à t.a. et sous N₂. Après 25 min, le mélange a été versé sur NaHSO₃ aq. à 10% (50 ml) et extrait à l'éther. La phase organique a été lavée successivement avec NaHCO₃ aq. sat. et NaCl aq. sat., et séchée sur Na₂SO₄ anhydre. La concentration sous vide a fourni le triméthyl silyl éther de l'intermédiaire désiré, qui a été dissous dans le THF (15 ml) et agité avec HCl aq. à 10% pendant 20 min. Re-extraction à l'éther, traitement et chromatographie [SiO₂ (100 g) ; éluant : cyclohexane/acétate d'éthyle 4:1], suivis de distillation au four à boules, ont permis d'obtenir la (Z)-3-(8'-hydroxyoct-1'-yl)-2-(2-pentényl)-1-cyclopentanone (trans/cis 13:1) sous forme d'une huile incolore (1,25 g ; rend. 78%).
P. éb. : 220-240° (bain) / 5,3 Pa
IR (CHCl₃) : 3440 (large), 3015, 2930, 2857, 1732, 1462 cm⁻¹
RMN (¹H, 360MHz, D₂O) : 4,96(t, J=7Hz, 3H) ; 1,20-2,40(24H) ; 3,63(t, J=7Hz, 2H); 5,25 (m, 1H) ; 5,42(m, 1H) δ ppm
RMN (¹³C) : 221,0(s), 133,5(d) ; 125,5(d) ; 62,8(t) ; 55,1(d) ; 41,1(d) ; 38,1(t) ; 34,7(t) ; 32,7(t) ; 29,8(t) ; 29,6(t) ; 29,4(t) ; 27,1(2t) ; 25,8(t) ; 25,4(t) ; 20,6(t) ; 14,2(q) δ ppm
SM : 280 (2,M⁺), 212(13), 151(36), 124(33), 109(15), 95(40), 83(100)

Du réactif de Jones (H₂CrO₄/H₂SO₄ aq. ; 4 ml d'une solution 2,5 M) a été ajouté goutte à goutte à une solution agitée de cette dernière cyclopentanone (1,1 g, 3,9 mmole ; trans/cis 13:1) dans l'acétone (20 ml) à 20°, sous N₂. Après encore 40 min à 22-24°, le mélange a été versé dans l'eau et extrait à l'éther. La phase organique a été lavée successivement avec H₂O, NaCl aq. à 10% et NH₄Cl aq. sat., séchée sur Na₂SO₄ anhydre et concentrée sous vide. L'huile jaune résultante (2,1 g) a été purifiée par chromatographie [SiO₂ (50 g) ; éluant : cyclohexane/acét. éthyle 1:1,5)] pour fournir l'acide (Z)-3-oxo-2-(2-pentényl)-1-cydopentaneoctanoïque désiré (trans/cis 13:1) sous forme d'une huile visqueuse jaune pâle (1 g ; rend. 87%).
IR (CHCl₃) : 3300 (large), 3020, 2931, 1732, 1710 cm⁻¹
RMN (¹H, 360MHz, D₂O) : 0,96(t, J=7Hz, 3H) ; 1,20-2,40(24H) ; 5,24(m, 1H) ; 5,42(m, 1H) δ ppm
RMN (¹³C) : 221,1(s) ; 179,3(s) ; 133,5(d) ; 125,5(d) ; 55,1(d) ; 41,1(d) ; 38,1(t) ; 34,7(t) ; 33,9(t) ; 29,6(t) ; 29,2(t) ; 29,0(t) ; 27,1(t) ; 27,0(t) ; 25,4(t) ; 24,7(t) ; 20,6(t) ; 14,2(q) δ ppm
SM : 294 (<0,5,M⁺), 226(1), 151(8), 124(16), 95(26), 83(100)

### Exemple 4

On a procédé selon la méthode générale II décrite dans l'Exemple 3, en utilisant les microorganismes cités au tableau ci-après. Dans ce tableau, les composés A, B, C et D sont ceux cités dans l'Exemple 3. Les résultats indiqués ont trait au produit obtenu après 24 h de réaction. Les rendements molaires sont indiqués en % par rapport à l'acide de départ ajouté, i.e. l'acide (Z)-3-oxo-2-(2-pentényl)-1-cyclopentaneoctanoïque (trans/cis 13:1,20 mg, ∼ 65 µmole).

Les résultats de ce tableau montrent que dans les cas des réactions avec *Rhodococcus rhodochorus, Arthrobacter petroleophagus* et *Aspergillus niger*, on n'observe essentiellement pas de résolution cinétique entre les énantiomères (+) et (-) du substrat, alors que dans tous les autres cas la quantité d'acide (+)-trans,(Z)-3-oxo-2-(2-pentényl)-1-cyclopentaneacétique formé (correspondant au composé A) est supérieure à celle de son énantiomère (-)-trans. Ceci indique donc que les microorganismes correspondants sont capables d'une résolution cinétique parmi les énantiomères du substrat. Par ailleurs, il ressort de ce tableau que cette résolution a lieu essentiellement lors de la conversion de l'acide (Z)-3-oxo-2-(2-pentényl)-1-cyclopentanebutanoïque (correspondant au composé B) en l'acide (Z)-3-oxo-2-(2-pentényl)-1-cyclopentaneacétique.

Les résultats d'expériences identiques effectuées à l'aide de *Cylindrocarpon candidum CBS 132,25, Arthrobacter atrocyanus DSN 20127*, *Streptomyces bacilliaris DSM 40598*, *Arthrobacter ureafaciens DSM 419* ou *Streptomyces rosechromogenus* semblent indiquer que ces microorganismes nécessitent un temps de réaction nettement supérieur à 24 h pour effectuer l'oxydation en β des substrats cités au tableau, dans les conditions de réaction décrites.

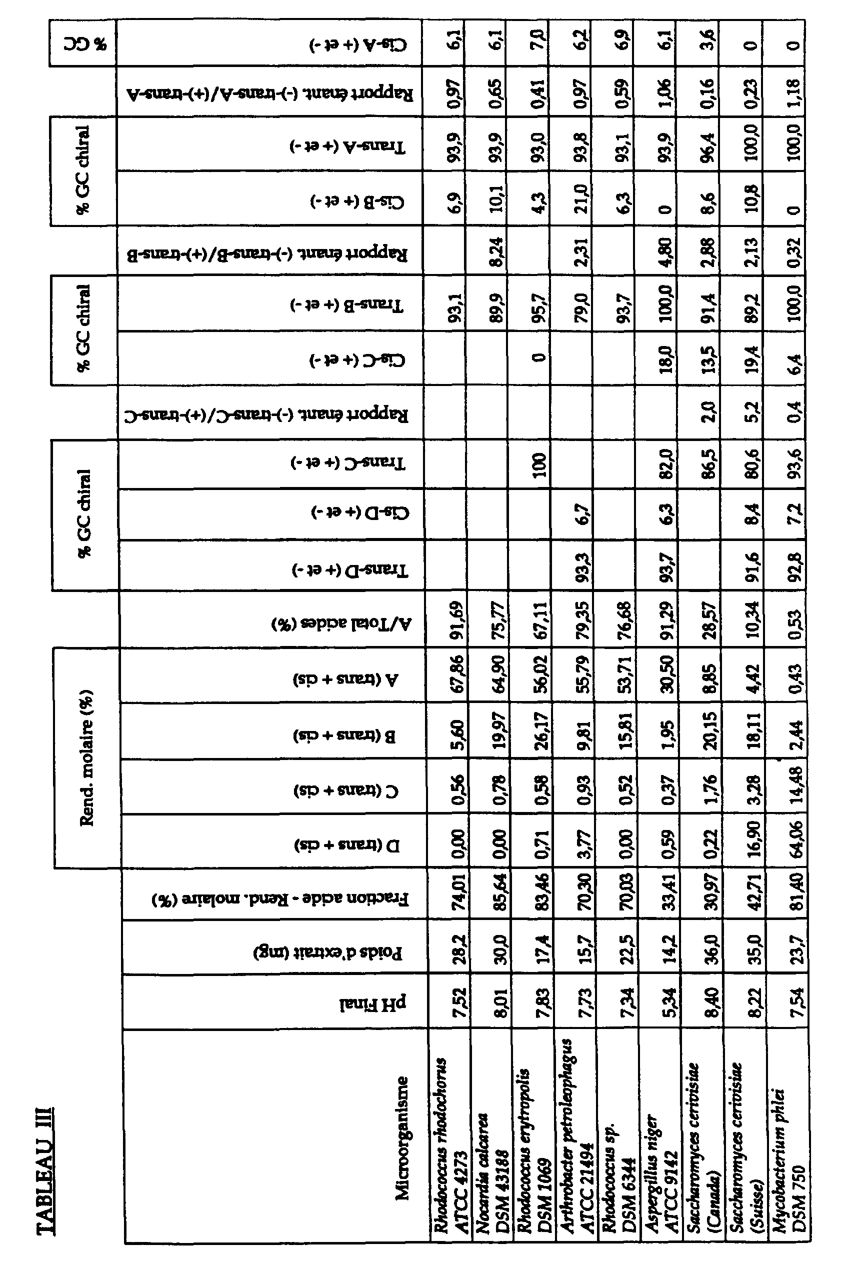

### Exemple 5

On a procédé selon la méthode générale I décrite dans l'Exemple 1, en ajoutant 100 mg d'acide 2-oxo-1-cyclopentanehexanoïque (obtenu par saponification avec NaOH de son ester éthylique disponible chez Janssen) à une suspension de *Saccharomyces cerevisiae*.

La fraction acide (38,6 mg) du produit de la réaction a été estéréfiée pour fournir un mélange d'esters contenant 25% en poids de l'ester méthylique de l'acide de départ, ainsi que les deux composés suivants :

### 2-oxo-1-cyclopentaneacétate de méthyle (12,9%)

- CG-SM :: 156 (41) [M⁺], 125 (100) [M-OCH₃⁺], 124 (97), 113 (34), 97 (47), 83 (54) [C₅H₆O+H⁺], 74 (48) [M-CH₂COOCH₃⁺], 59 (49)

### 2-oxo-1-cyclopentanebutyrate de méthyle (41,2%)

- CG-SM :: 184 (13) [M^{**+**}], 153 (19) [M-OCH₃⁺], 152 (53), 137 (18), 124 (28), 101 (4) [M-(CH₂)₃COOCH₃⁺], 97 (48), 84 (100) [C₅H₈O], 74 (47), 55 (38)

## Revendications

1. Procédé pour la préparation de cétones cycliques de formule 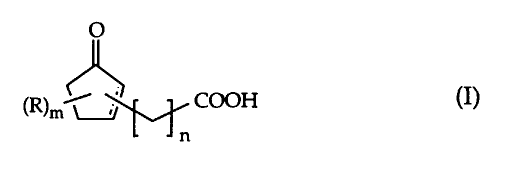 pouvant avoir une double liaison dans la position indiquée par la ligne pointillée et dans laquelle m représente un nombre entier de 0 à 3 et n un nombre entier de 0 à 10, le ou les symboles R représentent l'hydrogène ou des radicaux alkyles identiques ou différents, saturés ou insaturés, linéaires ou ramifiés, ayant de 1 à 6 atomes de carbone, chacun des groupes substituants pouvant se trouver dans n'importe quelle position libre du cycle, le procédé étant caractérisé en ce qu'on ajoute un substrat contenant un ou plusieurs dérivés cycliques carboxyliques de formule 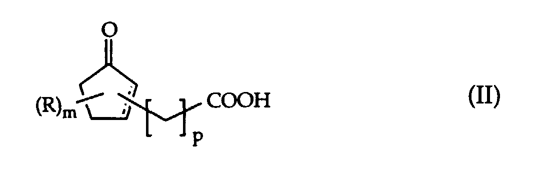 dans laquelle la ligne pointillée et les symboles R et m ont le sens indiqué à la formule (I), p ≥ n+2 et est défini comme étant un nombre entier pair lorsque n est pair et impair lorsque n est impair, à une culture d'un microorganisme capable d'effectuer l'oxydation en β de la chaîne d'acide gras desdits dérivés, pour fournir au moins une des cétones désirées, que l'on extrait ensuite du milieu réactionnel.

2. Procédé selon la revendication 1, caractérisé en ce qu'on ajoute un substrat contenant un ou plusieurs dérivés de formule 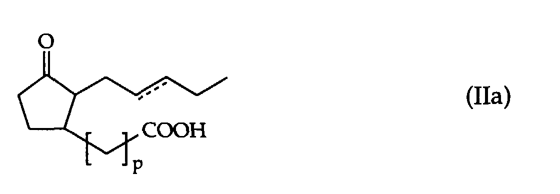 dans laquelle la ligne pointillée indique l'emplacement d'une liaison simple ou double et p ≥ n+2 et est impair.

3. Procédé selon la revendication 1, caractérisé en ce qu'on ajoute un substrat contenant un ou plusieurs dérivés de formule 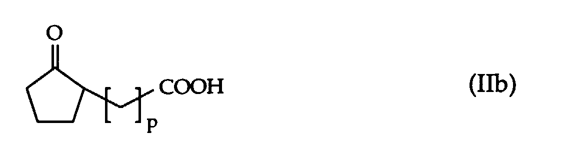 dans laquelle p a le sens indiqué à la formule (II).

4. Procédé selon l'une des revendications 1 à 3, caractérisé en ce que le microorganisme est du genre *Saccharomyces* ou *Rhodococcus*.

5. Procédé selon l'une des revendications 1 à 3, caractérisé en ce que le microorganisme est choisi dans le groupe constitué par *Rhodococcus rhodochorus, Rhodococcus erytropolis, Rhodococcus sp., Nocardia calcarea, Arthrobacter petroleophagus, Arthrobacter artrocyanus, Arthrobacter ureafaciens, Aspergillus niger, Saccharomyces cerivisae, Mycobacterium phlei, Streptomyces viridosporus, Streptomyces rosechromogenus, Streptomyces bacilliaris, Cylindrocarpon candidum, Escherichia coli, Hansenula polymorpha, Pseudomonas Sp., Serratia marcesens et Aspergillus oryzae.*

6. Procédé selon la revendication 5, caractérisé en ce qu'on ajoute de l'acide (Z)-3-oxo-2-(2-pentényl)-1-cyclopentaneoctanoïque à une culture d'un microorganisme choisi dans le groupe constitué par *Rhodococcus rhodochorus, Rhodococcus erytropolis, Rhodococcus sp., Nocardia calcarea* et *Arthrobacter petroleophagus*, pour obtenir essentiellement de l'acide (Z)-3-oxo-2-(2-pentényl)-1-cyclopentaneacétique.

7. Procédé selon la revendication 5, caractérisé en ce qu'on ajoute de l'acide 3-oxo-2-pentyl-1-cyclopentaneoctanoïque à une culture d'un microorganisme choisi dans le groupe constitué par *Serratia marcesens* et *Aspergillus niger*, pour obtenir essentiellement de l'acide 3-oxo-2-pentyl-1-cyclopentaneacétique.

8. Procédé selon l'une des revendications précédentes, caractérisé en ce qu'il comprend lestérification subséquente du produit obtenu, pour former l'ester correspondant.

9. Procédé selon l'une des revendications 1 à 7, caractérisé en ce que l'addition du substrat à la culture du microorganisme a lieu dans un milieu dépourvu de toute autre source nutritive.

## Claims

1. Process for the preparation of cyclic ketones of formula 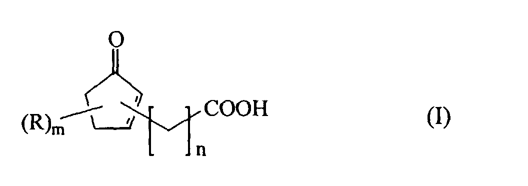 optionally having a double bond in the position indicated by the dotted line and wherein m represents an integer from 0 to 3 and n an integer from 0 to 10, each of the symbols R, which can be identical or different, stands for hydrogen or for a saturated or unsaturated, linear or branched, alkyl radical having 1 to 6 carbon atoms, and each of the substituent groups can be located in any available positions of the ring, the process being characterized in that a substrate containing one or several cyclic carboxylic derivatives of formula 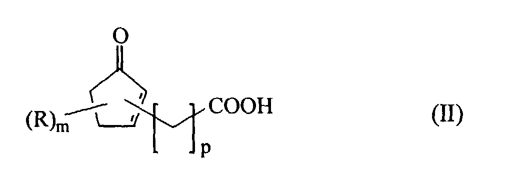 wherein the dotted line and the symbols R and m have the meaning indicated in formula (I), p ≥ n+2 and is defined as being an even integer when n is even and an odd number when n is odd, is added to a culture of a microorganism capable of β-oxidising the fatty acid chain of said derivatives to form at least one of the desired ketones, which is then extracted from the reaction medium.

2. A process according to claim 1, characterized in that there is added a substrate containing one or several derivatives of formula 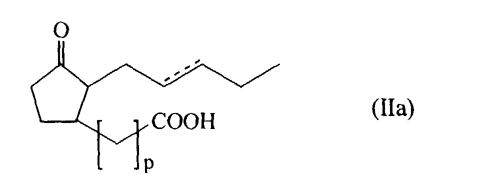 wherein the dotted line indicates the position of a single or double bond and p≥ n+2 and is odd.

3. A process according to claim 1, characterized in that there is added a substrate containing one or several derivatives of formula 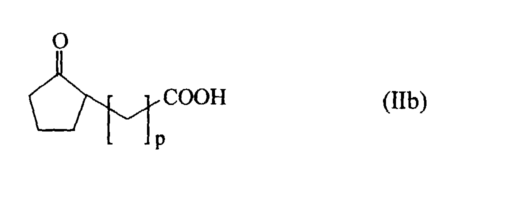 wherein p has the meaning indicated in formula (II).

4. A process according to any one of claims 1 to 3, characterized in that the microorganism is of the *Saccharomyces* or *Rhodococcus* genus.

5. A process according to any one of claims 1 to 3, characterized in that the microorganism is selected from the group consisting of *Rhodococcus rhodochorus, Rhodococcus ergropolis, Rhodococcus sp., Nocardia calcarea, Arthrobacter petroleophagus, Arthrobacter artrocyanus, Arthrobacter ureafaciens, Aspergillus niger, Saccharomyces cerivisae, Mycobacterium phlei, Streptomyces viridosporus, Streptomyces rosechromogenus, Streptomyces bacilliaris, Cylindrocarpon candidum, Escherichia coli, Hansenula polymorpha, Pseudomonas Sp., Serratia marcesens* and Aspergillus oryzae.

6. A process according to claim 5, characterized in that (Z)-3-oxo-2-(2-pentenyl)-1-cyclopentaneoctanoic acid is added to a culture of a microorganism selected from the group consisting of *Rhodococcus rhodochorus, Rhodococcus erytropolis, Rhodococcus sp., Nocardia calcarea* and *Arthrobacter petroleophagus* to form essentially (Z)-3-oxo-2-(2-pentenyl)-1-cyclopentaneacetic acid.

7. A process according to claim 5, characterized in that 3-oxo-2-pentyl-1-cyclopentaneoctanoaic acid is added to a culture of a microorganism selected from the group consisting of *Serratia marcesens* and *Aspergillus niger* to form essentially 3-oxo-2-pentyl-1-cyclopentaneacetic acid.

8. A process according to any one of the preceding claims, characterized in that it comprises the subsequent esterification of the product obtained, to form the corresponding ester.

9. A process according to any one of claims 1 to 7, characterized in that the addition of the substrate to the microorganism culture is performed in a media devoid of any other nutritive materials.

## Patentansprüche

1. Verfahren zur Herstellung von cyclischen Ketonen mit der Formel 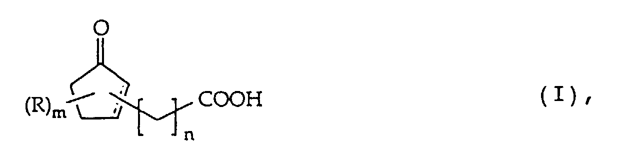 die eine Doppelbindung in der durch die gestrichelte Linie angedeuteten Position aufweisen kann, und in der m für eine ganze Zahl von 0 bis 3 und n für eine ganze Zahl von 0 bis 10 steht, wobei das Symbol bzw. die Symbole R für Wasserstoff oder Alkylreste steht/stehen, die gleich oder voneinander verschieden, gesättigt oder ungesättigt, geradlinig oder verzweigt sind, 1 bis 6 Kohlenstoffatome aufweisen, und jede der Substituentengruppen sich in einer beliebigen freien Position des Rings befinden kann, wobei das Verfahren dadurch gekennzeichnet ist, daß ein Substrat, das ein oder mehrere cyclische Carboxylderivate mit der Formel 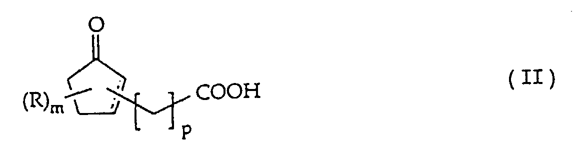 enthält, in der die gestrichelte Linie und die Symbole R und m die für Formel (I) angegebene Bedeutung haben, p ≥ n+2 und als ganze gerade Zahl definiert ist, wenn n geradzahlig ist, und als ungerade, wenn n ungeradzahlig ist, einer Kultur eines Mikroorganismus zugegeben wird, der in der Lage ist, die Oxidation in der β-Position der Fettsäurekette der Derivate zu bewirken, um mindestens eines der gewünschten Ketone zu liefern, das anschließend aus dem Reaktionsmedium extrahiert wird.

2. Verfahren nach Anspruch 1, dadurch gekennzeichnet, daß ein Substrat zugegeben wird, das ein oder mehrere Derivate mit der Formel 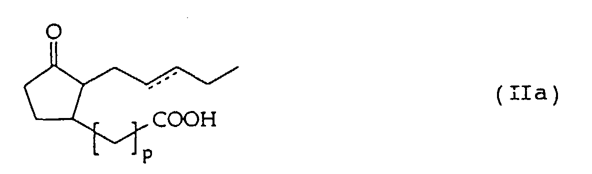 enthält, in der die gestrichelte Linie die Stelle einer Einfach- oder Doppelbindung angibt, und p ≥ n+2 und ungeradzahlig ist.

3. Verfahren nach Anspruch 1, dadurch gekennzeichnet, daß ein Substrat zugegeben wird, das ein oder mehrere Derivate mit der Formel 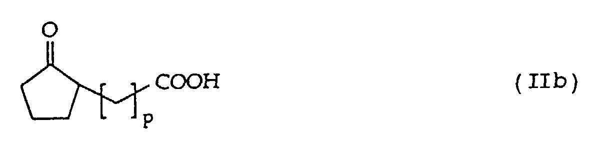 enthält, in der p die für Formel (II) angegebene Bedeutung hat.

4. Verfahren nach einem der Ansprüche 1 bis 3, dadurch gekennzeichnet, daß der Mikroorganismus von der Gattung *Saccharomyces* oder *Rhodococcus* ist.

5. Verfahren nach einem der Ansprüche 1 bis 3, dadurch gekennzeichnet, daß der Mikroorganismus aus der Gruppe ausgewählt ist, die besteht aus *Rhodococcus rhodochorus, Rhodococcus erytropolis, Rhodococcus sp., Nocardia calcarea, Arthrobacter petroleophagus, Arthrobacter artrocyanus, Arthrobacter ureafaciens,* *Aspergillus niger, Saccharomyces cerivisae, Mycobacterium phlei, Streptomyces viridosporus, Streptomyces rosechromogenus, Streptomyces bacilliaris, Cylindrocarpon candidum, Escherichia coli, Hansenula polymorpha, Pseudomonas Sp., Serratia marcesens* und *Aspergillus oryzae.*

6. Verfahren nach Anspruch 5, dadurch gekennzeichnet, daß (Z)-3-Oxo-2-(2-pentenyl)-1-cyclopentanoctansäure einer Kultur eines Mikroorganismus zugegeben wird, der aus der aus *Rhodococcus rhodochorus, Rhodococcus erytropolis*, *Rhodococcus sp., Nocardia calcarea* und *Arthrobacter petroleophagus* bestehenden Gruppe ausgewählt ist, um im wesentlichen (Z)-3-Oxo-2-(2-pentenyl)-1-cyclopentanessigsäure zu erhalten.

7. Verfahren nach Anspruch 5, dadurch gekennzeichnet, daß 3-Oxo-2-pentyl-1-cyclopentanoctansäure einer Kultur eines Mikroorganismus zugegeben wird, der aus der aus *Serratia marcesens* und *Aspergillus niger* bestehenden Gruppe ausgewählt ist, um im wesentlichen 3-Oxo-2-pentyl-1-cyclopentanessigsäure zu erhalten.

8. Verfahren nach einem der vorhergehenden Ansprüche, dadurch gekennzeichnet, daß es die anschließende Veresterung des erhaltenen Produktes zur Bildung des entsprechenden Esters aufweist.

9. Verfahren nach einem der Ansprüche 1 bis 7, dadurch gekennzeichnet, daß die Zugabe des Substrats zur Kultur des Mikroorganismus in einem Medium stattfindet, das frei von jeglicher weiteren Nahrungsquelle ist.
